# EUROPEAN PATENT SPECIFICATION

(11) **EP 3 337 444 B1**
(45) Date of publication and mention of the grant of the patent: **07.10.2020**
(21) Application number: 16837737.2
(22) Date of filing: 17.08.2016
(51) Int. Cl.: A61K 8/44, A61Q 5/00, A61Q 5/06, A61Q 5/12, A61K 8/81

(54) **SYNERGISTIC CONDITIONING AND/OR STYLING COMPOSITIONS COMPRISING POLYQUATERNIUM-37 AND POLYAPTAC**
SYNERGISTISCHE BALSAM- UND/ODER HAARFORMUNGSZUSAMMENSETZUNGEN MIT POLYQUATERNIUM-37 UND POLYAPTAC
COMPOSITIONS SYNERGIQUES DE TRAITEMENT ET/OU DE MISE EN FORME COMPRENANT DU POLYQUATERNIUM-37 ET DU POLYAPTAC

(30) Priority: 17.08.2015 US 201562205959 P
(43) Date of publication of application: 27.06.2018
(73) Proprietor: Hercules LLC, Wilmington, DE 19808 (US)
(72) Inventor: KROON, Gijsbert, 3371BW Hardinxveld- Giessendam (NL); NUUTINEN, Tuttu Maria, 2613 BT Delft (NL); NGUYEN, Thi Bich Van, 3317VM Dordrecht (NL)
(74) Representative: Kutzenberger Wolff & Partner
(86) International application number: PCT/US2016/047300
(87) International publication number: WO 2017/031180

(56) References cited:
- EP-A1- 1 787 633
- WO-A1-2014/071354
- WO-A1-2014/071354
- WO-A1-2016/062483
- WO-A2-02/083073
- US-A- 6 110 451
- US-A1- 2013 209 388
- IP: "Researchers Submit Patent Application, "Synergistic Conditioning And/Or Styling Compositions Comprising Polyquaternium-37 And Polyaptac", for Approval (USPTO 20180243199)", HEALTH AND MEDICINE WEEK, 17 June 2015 (2015-06-17), page 8055, XP055539033, US ISSN: 1531-6459
- DROVETSKAYA. TV ET AL.: 'New high-charge density hydrophobically modified cationic HEC polymers for improved co-deposition of benefit agents and serious conditioning for problem hair.' JOURNAL OF COSMETIC SCIENCE vol. 58, August 2007, pages 421 - 434, XP055235466

## Description

### FIELD OF THE INVENTION

The present application relates to a personal care composition, and, more particularly, to a personal care composition comprising a conditioning and/or styling copolymer for a keratin substrate of hair and/or skin origin, as defined in the appended claims.

### BACKGROUND OF THE INVENTION

A particular challenge for a personal care product developed for damaged hair is to condition hair damaged by heat, or damaged mechanically or chemically through bleaching or coloring the hair. Consumers desire to have existing damage to the hair be repaired and to prevent further damage to the hair from occurring. It is desired that conditioned hair be easily manageable, feel-smooth, and be easy to detangle. However, if hair is over-conditioned, the hair is perceived as being weighed down.

Silicones are traditionally used in hair care applications to provide conditioning properties such as ease of combing and detangling, color retention, smoothness and hydrophobicity. Silicones function by forming a film on the hair surface and depending on the nature of the silicone they are either self-deposited or they require additional cationic ingredients for deposition. It is desired to formulate conditioning systems having reduced or no silicones while still providing silicone's benefits. The conditioning composition presented in the invention provides improved or similar properties to silicones and can also be used in the applications formulated without silicones.

The IP.com prior art database technical disclosure no. 000242076 reveals a personal care conditioning and/or styling composition for a keratin substrate comprising: (i) about 0.1 *wt.* % to about 20 *wt.* % of PolyAPTAC polymer; (ii) about 0.1 *wt.* % to 99.9 wt. % of water; (iii) at least one cosmetically acceptable excipient; (iv) optionally, about 0.1 *wt.* % to about 1 *wt.* % of one or more natural or synthetic based monomer or polymer selected from the group consisting of anionic, cationic, non-ionic, amphoteric, hydrophobic, or zwitterionic in nature; and (v) optionally, at least one personal care active ingredient. Other relevant prior arts in this regard would include US8597623, US2015105500 and US6110451.

### SUMMARY OF THE INVENTION

The primary objective of the present application is to provide a personal care conditioning and/or styling synergistic composition for a keratin substrate comprising:
i. 0.1 wt. % to 20 wt. % of poly(acrylamido-N-propyltrimethylammonium chloride) (PolyAPTAC) polymer; wherein the average molecular weight of PolyAPTAC is in the range of from 100,000 to 1,000,000 g/mol;
ii. 0.1 wt. % to 5 wt. % of Polyquaternium-37;
iii. 0.1 wt. % to 5 wt. % of at least one cationic surfactants;
iv. 0.1 wt. % to 99.59 wt. % of water; and
v. 0.01 wt. % to 20 wt. % of at least one cosmetically acceptable excipient, wherein the composition is capable of providing immediate and prolonged benefit to a desired keratin substrate which is hair or skin.

Another objective of the present application is to provide a synergistic conditioning and/or styling composition comprising PolyAPTAC and at least one quaternary ammonium compound.

One another objective of the present application is to provide a method for treating or fixing regular or damaged keratin substrate comprising contacting said keratin substrate with an effective amount of personal care composition of claim 1.

### BRIEF DESCRIPTION OF THE FIGURES

Further embodiments of the present invention can be understood with the appended figures.
**FIG. 1** **&** **3** shows Wet comb energy - Texture Analysis of samples
**FIG. 2** shows Rheology Profile Viscosity *vs*. Shear stress of samples

### DETAILED DESCRIPTION OF THE INVENTION

What is described herein is a personal care conditioning and/or styling synergistic composition for a keratin substrate comprising: (i) about 0.1 *wt.* % to about 20 *wt.* % of PolyAPTAC polymer; (ii) about 0.1 *wt.* % to about 5 *wt.* % of Polyquaternium-37; (iii) about 0.1 wt. % to about 5 *wt.* % of at least one cationic surfactants; (iv) about 0.1 *wt.* % to 99.9 *wt*. % of water; and (v) at least one cosmetically acceptable excipient and wherein the composition is capable of providing immediate and prolonged benefit to a desired keratin substrate which is hair or skin.

One important aspect is to provide a synergistic conditioning and/or styling composition comprising PolyAPTAC and at least one quaternary ammonium compound, wherein, the preferred quaternary ammonium compound is polyquaternium compounds that are known for a person skilled in the relevant art and are capable of providing synergy with PolyAPTAC polymer. However, most preferred Polyquaternium compound for the present application is PQ-37.

Further, it is contemplated to employ suitable polyquaternium type of cationic polymers for the present application would include but not limited to Polyquaternium 4, Polyquaternium 5, Polyquaternium 6, Polyquaternium 7, Polyquaternium 10, Polyquaternium 11, Polyquaternium 15, Polyquaternium 16, Polyquaternium 22, Polyquaternium 28, Polyquaternium 32, Polyquaternium 39, Polyquaternium 46, Polyquaternium 47, Polyquaternium 53, Polyquaternium 55, Polyquaternium 67, Polyquaternium 87, polyquaternium-90, polyquaternium-91, polyquaternium-92, polyquaternium-94, Polyquaternium- 95, and/or Polyquaternium-96. Other polymers known by their CTFA category name "Quaternium" are suitable for the present application would include but not limited to Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

Other cationic polymers that may be used within the context of the invention are cationic proteins or hydrolyzed cationic proteins, polyalkyleneimines such as polyethyleneimines, polymers containing vinyl pyridine or vinyl pyridinium units, condensates of polyamines and epichlorhydrins, quaternary polyurethanes, and derivatives of chitin.

Suitable cationic surfactants for the present application is selected from the group including but not limited to, primary amines, secondary amines, tertiary amines, quaternary amines, alkanolamines, mono-alkyl alkanolamines, di-alkyl alkanolamines, tri-alkyl alkanolamines, alkyl mono alkanolamines, alkyl di-alkanolamines, alkylamines, mono-alkyl amines, di-alkyl amines, tri-alkylamines, alkoxylated amines, alkyl and aryl amine alkoxylates, methoxylated alkylamines, ethoxylated alkylamines, alkoxylated alkanolamines, alkyl alkanolamines, alkoxylated ethylene diamine derivatives, alkyl/aryl/arylalkyl amine oxides. Preferred cationic surfactants of the present invention include, but are not limited to, (a) alkyl alkanolamines; and (b) alkyl tertiary amines. Additional information on useful cationic surfactants for the purpose of present invention is set forth in McCutcheon's Detergents and Emulsifiers, North American Ed., 1982 and Kirk-Othmer, Encyclopedia of Chemical Technology, 3rd Ed., Vol. 22, pp. 346-387*,* the contents of which are incorporated herein by reference.

As used herein, the term "cosmetically acceptable excipient" means any ingredient/compound or mixture of ingredients/compounds or compositions that are typically employed to produce other desirable effects in personal care compositions. The preferred cosmetically acceptable excipients include but not limited to preservatives, antioxidants, chelating agents, sunscreen agents, proteins, amino acids, vitamins, dyes, hair coloring agents, plant extracts, plant derivatives, plant tissue extracts, plant seed extracts, plant oils, botanicals, botanical extracts, humectants, fragrances, perfumes, oils, emollients, lubricants, butters, penetrants, thickeners, viscosity modifiers, thickeners, polymers, resins, hair fixatives, film formers, surfactants, detergents, emulsifiers, opacifying agents, volatiles, propellants, liquid vehicles, carriers, salts, pH adjusting agents, neutralizing agents, buffers, hair conditioning agents, anti-static agents, anti-frizz agents, anti-dandruff agents, hair waving agents, hair straightening agents, relaxers, absorbents, fatty substances, gelling agents, moisturizers, hydrophilic or lipophilic active agent, preserving agents, fillers, dyestuffs, reducing agents, cosmetic oils, perfumes, liquid vehicles, solvents, carriers, silicones, and combinations thereof. The preferred range of said excipient is from about 0.1 *wt.* % to 99.9 *wt*. % of the total composition. Other ranges would include about 0.01 *wt.* % to 10 *wt.* %, about 10.1 *wt.* % to about 20 *wt.* %, about 20.1 *wt.* % to about 30 *wt.* %, about 30.1 *wt.* % to about 40 *wt.* %, about 40.1 *wt.* % to about 50 *wt.* %, about 50.1 *wt.* % to about 60 *wt.* %, about 60.1 *wt.* % to about 70 *wt.* %, about 70.1 *wt.* % to about 80 *wt.* %, about 80.1 *wt.* % to about 90 *wt.* %, and about 90.1 wt. % to about 99.0 wt. %. The preferred cosmetically acceptable excipients for the purposes of present application can be selected from published application WO2014071354.

The personal care composition of present application can be an appropriate product selected from the group consisting of hair-care products, shampoos, hair conditioners, 2 in 1 shampoos, leave in and rinse off conditioners, hair treatments including intensive treatments, styling and treating hair compositions, hair perming products, hair straighteners, hair relaxants, hair sprays and lacquers, permanent hair dyeing systems, hair styling mousses, hair gels, semi-permanent hair dyeing systems, temporary hair dyeing systems, hair bleaching agents, permanent hair wave systems, hair setting formulations, non-coloring hair preparations, hair-frizz-control gels, hair leave-in conditioners, hair pomades, hair de-tangling products, hair fixatives, hair conditioning mists, hair care pump sprays and other non-aerosol sprays, skin-care products, hair cuticle coats, skin care moisturizing mists, skin wipes, pore skin wipes, pore cleaners, blemish reducers, skin exfoliators, skin desquamation enhancers, skin towelettes, skin protection ointments, skin powders, skin pads, paste masks and muds, face masks, facial cleansing products, anti-acne preparations, bath products, shower products, liquid soaps, bar soaps, body oils, body lotions, body gels, body and hand preparations, face and body washes, bath salts, bath and body milks, foam baths, synthetic and non-synthetic soap bars, hand liquids, shaving lotions, shaving and aftershave preparations, pre-shaves and pre-electric shaves, nail varnishes, nail polish, nail polish remover, nail creams and lotions, cuticle softeners, nail conditioners, eye shadows, mascaras, eye liners, eye shadows, blushes, makeup, eye shadow sticks, baby lotions, baby baths and shampoos, baby conditioners, fragrances and/or odoriferous ingredients consisting preparations, dentifrices, deodorizing and antiperspirant preparations, decorative preparations, light protection formulations, treatment creams, lipsticks, dry and moist make-up, rouge, powders, depilatory agents, sun care products, compositions comprising UV blockers or UV protectors, anti-aging products, foundations, face powders, moisturizing preparations, tanning preparations, nose strips, make-up removers, cold creams, mousses, shower gels, personal care rinse-off products, gels, scrubbing cleansers, astringents, lip balms, lip glosses, anhydrous creams and lotions, oil/water, water/oil, multiple and macro and micro emulsions, water-resistant creams and lotions, mouth-washes, massage oils, toothpastes, clear gels and sticks, ointment bases, topical wound-healing products, aerosol talc, barrier sprays, vitamin, herbal-extract preparations, and/or controlled-release personal care products.

The personal care composition of present invention can be formulated in several required forms according to their necessity, and the non-limiting forms include emulsion, lotion, gel, vesicle dispersion, paste, cream, solid stick, mousse, shampoo, spray, balm, wipe, milk, foam, jellies, liquid, tonics, and/or enamel.

According to one embodiment of the present application, the ratio between PolyAPTAC and PQ-37 to obtain desired synergy is from about 1:10 to about 10:1, however, the other preferred ratio of the present application is 1:1, 1:2, 2:1, 1:3, 3:1, 1:4, 4:1, 1:5, 5:1, 1:6, 6:1, 1:7, 7:1, 1:8, 8:1, 1:9, or 9:1. Most preferred ratio of the present application is from about 1: about 1.

A method for treating or fixing regular or damaged keratin substrate comprising contacting said keratin substrate with an effective amount of personal care composition of claim 1 comprising: (i) about 0.1 *wt.* % to about 20 *wt.* % of PolyAPTAC polymer; (ii) about 0.1 *wt.* % to about 5 *wt.* % of Polyquaternium-37; (iii) about 0.1 wt. % to about 5 *wt.* % of at least one cationic surfactants; (iv) about 0.1 *wt.* % to 99.9 wt. % of water; and (v) at least one cosmetically acceptable excipient.

A method for washing or caring a keratin substrate comprising applying an effective amount of composition of claim 1 comprising: (i) about 0.1 *wt.* % to about 20 *wt.* % of PolyAPTAC polymer; (ii) about 0.1 *wt.* % to about 5 *wt.* % of Polyquaternium-37; (iii) about 0.1 wt. % to about 5 *wt.* % of at least one cationic surfactants; (iv) about 0.1 *wt.* % to 99.9 wt. % of water; and (v) at least one cosmetically acceptable excipient.

The examples are presented to illustrate the invention, parts and percentages being by weight, unless otherwise indicated.

Further, certain aspects of the present invention are illustrated in detail by way of the following examples. The examples are given herein for illustration of the invention and are not intended to be limiting thereof.

### Examples:

Phase A, B, C and D were prepared as per the w/w compositions of **Tables 1 & 2.** Phase A is first prepared and ingredients mixed together until homogenous at 70-80°C. Phase B is separately formulated at 70-80°C and ingredients mixed together until homogenous - then added to Phase A and homogenized with a high shear mixer and cooled down to approx. 40°C. Then Phase C & D ingredients added & mixed until homogenous.

Each of these phases were used to prepare Examples a, b, c, d, e, f, g, h, i, j, k, l and m, wherein sample "m" is formulated where the poly APTAC polymer was added in the Phase C as "cold phase" to further improve the performance of the hair care product, and samples "f" and "l" are mix PQ37 and Poly APTAC polymer without additional rheology modifiers added.

**Table 1: Hair Care Compositions**

| **Ingredients** | **% w/w** | | | | | |
|---|---|---|---|---|---|---|
| **Phase A** | **a** | **b** | **c** | **d** | **e** | **f** |
| Deionized water Cetyl Hydroxyethylcellulose | ad100% - | ad100% | ad100% - | ad100% - | ad100% 0.10 | ad100% - |
| Hydroxyethylcellulose | 1.00 | 1.00 | 1.00 | 1.00 | - | - |
| Polyacrylamidopropyltrimonium Chloride | - | 5.00 | 1.25 | 2.50 | 1.25 | 1.25 |

| **Phase B** | | | | | | |
|---|---|---|---|---|---|---|
| Polyquaternium-37 (and) Propyleneglycol Dicaprylate/Dicaprate (and) PPG-1 Trideceth-6 | 2.00 | - | 0.50 | 1.00 | 0.50 | 0.50 |
| Cationic Surfactant (Steramidopropyldimethylamine) | 0.70 | 0.70 | 0.70 | 0.70 | 0.70 | 0.70 |
| Cetearyl Alcohol | 2.50 | 2.50 | 2.50 | 2.50 | 2.50 | 2.50 |

| **Phase C** | | | | | | |
|---|---|---|---|---|---|---|
| Benzyl Alcohol (and) Aqua (and) Sodium Benzoate (and) Potassium Sorbate | 0.30 | 0.30 | 0.30 | 0.30 | 0.30 | 0.30 |

| **Phase D** | | | | | | |
|---|---|---|---|---|---|---|
| Citric Acid | To pH 4.0-4.5 | To pH 4.0-4.5 | To pH 4.0-4.5 | To pH 4.0-4.5 | To pH 4.0-4.5 | To pH 4.0 -4.5 |

**Table 2: Hair Care Compositions**

| **Ingredients** | **% w/w** | | | | | | |
|---|---|---|---|---|---|---|---|
| **Phase A** | **g** | **h** | **i** | **j** | **k** | **l** | **m** |
| Deionized water | ad100% | ad100% | ad100% | ad100% | ad100% | ad100% | ad100% |
| Disodium EDTA | 0.1 | 0.10 | 0.1 | 0.1 | 0.10 | 0.10 | 0.10 |
| Cetyl Hydroxyethylcellulose | - | - | - | - | 0.10 | - | 0.10 |
| Hydroxyethylcellulose | - | 1.00 | 1.00 | 1.00 | - | - | - |
| Glycerin | 2.00 | 2.00 | 2.00 | 2.00 | 2.00 | 2.00 | 2.00 |
| Polyacrylamidopropyltrimonium Chloride | - | 5.00 | 1.25 | 2.50 | 1.25 | 1.25 | - |

| **Phase B** | | | | | | | |
|---|---|---|---|---|---|---|---|
| Polyquaternium-37 (and) Propyleneglycol Dicaprylate/Dicaprate (and) PPG-1 Trideceth-6 | 2.00 | - | 0.50 | 1.00 | 0.50 | 0.50 | 0.50 |
| Cetrimonium Chloride Behenyl Alcohol (and) Cetearyl Alcohol | 5.00 | 5.00 | 5.00 | 5.00 | 5.00 | 5.00 | 5.00 |
| (and) Hydroxyethyl Cetearylamidopropyldimonium Chloride | 2.50 | 2.50 | 2.50 | 2.50 | 2.50 | 2.50 | 2.50 |
| Cetearyl Alcohol | 6.00 | 6.00 | 6.00 | 6.00 | 6.00 | 6.00 | 6.00 |
| Glyceryl Stearate | 1.00 | 1.00 | 1.00 | 1.00 | 1.00 | 1.00 | 1.00 |

| **Phase C** | | | | | | | |
|---|---|---|---|---|---|---|---|
| Polyacrylamidopropyltrimonium Chloride | - | - | - | - | - | - | 1.25 |
| Dimethiconol (and) TEA-Dodecylbenzenesulfonate | 2.50 | 2.50 | 2.50 | 2.50 | 2.50 | 2.50 | 2.50 |
| Amodimethicone/Morpholinomethyl Silsesquioxane Copolymer (and) Trideceth-5 (and) Glycerin | 2.50 | 2.50 | 2.50 | 2.50 | 2.50 | 2.50 | 2.50 |
| Perfume | 0.03 | 0.03 | 0.03 | 0.03 | 0.03 | 0.03 | 0.03 |
| Benzyl Alcohol (and) Aqua (and) Sodium Benzoate (and) Potassium Sorbate | 0.30 | 0.30 | 0.30 | 0.30 | 0.30 | 0.30 | 0.30 |

| **Phase D** | | | | | | | |
|---|---|---|---|---|---|---|---|
| Citric Acid | To pH 4.0-4.5 | To pH 4.0-4.5 | To pH 4.0-4.5 | To pH 4.0-4.5 | To pH 4.0-4.5 | To pH 4.0 -4.5 | To pH 4.0-4.5 |

### Wet Combability Study

The PolyAPTAC polymer was tested as a 1 wt. % in 2.5% fatty alcohol and 0.7 wt. % Cationic surfactants (Example "**b**" of Table 1). As a reference 1 wt. % Polyquaternium-37 in 2.5 wt. % fatty alcohols and 0.7 wt. % cationic surfactants was used (Example "**a**" of Table 1). To study a combination of PolyAPTAC and Polyquaternium-37 two examples were used. Example "**c**" consists of 0.25 wt. % PolyAPTAC polymer and 0.25 wt. % Polyquaternium-37 polymer in 2.5% fatty alcohol and 0.7% Cationic surfactants. Example "**d**" consists of 0.5 wt. % PolyAPTAC polymer and 0.5 wt. % Polyquaternium-37 polymer in 2.5 wt. % fatty alcohol and 0.7 wt. % Cationic surfactants. The conditioner was applied on damaged hair (0.2 grams per gram of bleached hair, 1 hour bleached) and wet comb energies were measured with Texture Analyzer after the rinse with water and after 1, 3 and 5 times of washing with 0.1 grams per gram hair shampoo (shampoo: 12/2 SLES/CAPB). The test results are provided in **Table 3:**

**Table 3: Wet comb energies**

| **Example** | **Wet Comb Energy rinse-off conditioner (gf-mm)** | **Wet Comb Energy 1 wash (gf-mm)** | **Wet Comb Energy 3 washes (gf-mm)** | **Wet Comb Energy 5 washes (gf-mm)** |
|---|---|---|---|---|
| **a** | 3839 | 6699 | 10,178 | 21,688 |
| **b** | 7306 | 3848 | 8847 | 17,247 |
| **c** | 5108 | 6570 | 12,714 | 16,629 |
| **d** | 4189 | 6211 | 7708 | 10,596 |
| **e** | 5436 | 8594 | 15,819 | 17,804 |

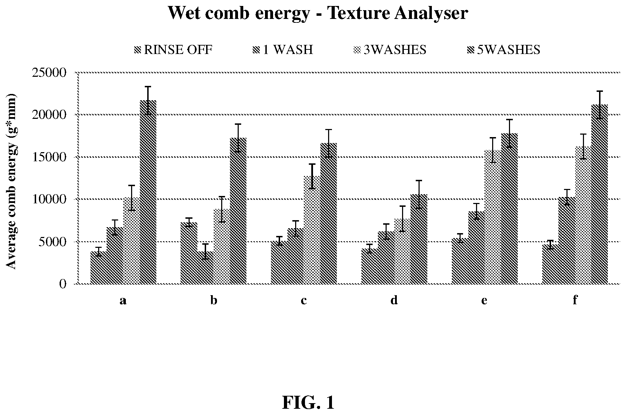

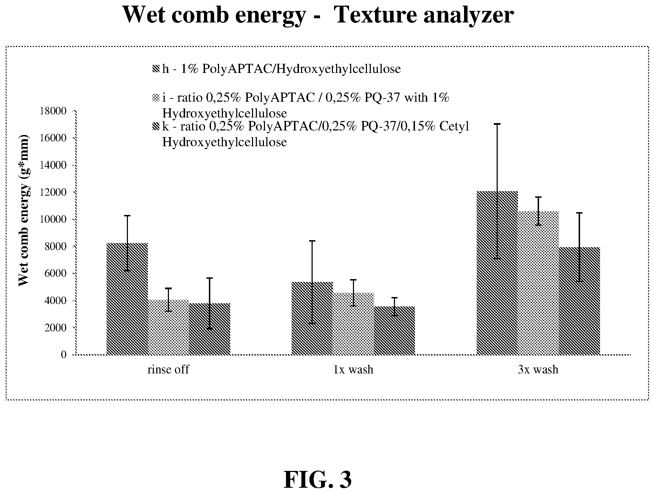

From the above results presented in **Table 3,** it was evident that a PolyAPTAC polymer when used in combination with Polyquaternium-37 provides better performance compared to the conditioning polymers alone. There is a clear synergy between the polymers resulting lower wet comb energy values. As seen in Table 3, the wet combing energy after 5 washes (gf-mm) was 21,688 for example "a" (formulated with 1 wt. % Polyquaternium-37), 17,247 for example "b" (formulated with 1 wt. % PolyAPTAC polymer) and only 10,596 for example "d" (formulated with combination of 0.5 wt. % Polyquaternium-37 and 0.5 wt. % PolyAPTAC polymer). These examples are clear indication of providing additional long lasting conditioning benefits when combining both Polyquaternium-37 and PolyAPTAC polymer. **Figure 1** provide a detail comparison of each sample, and wherein, "each data values are the average of 5 measurements done on 3 separated hair tresses (so an average of 15 measurements each). The error bar represents the SD of the average value of each hair tress. **Figure 3** represents the wet combing values for formulations h, i, k as expressed in **Table 2** showing good conditioning performances in particular when combining PolyAPTAC polymer and PQ37.

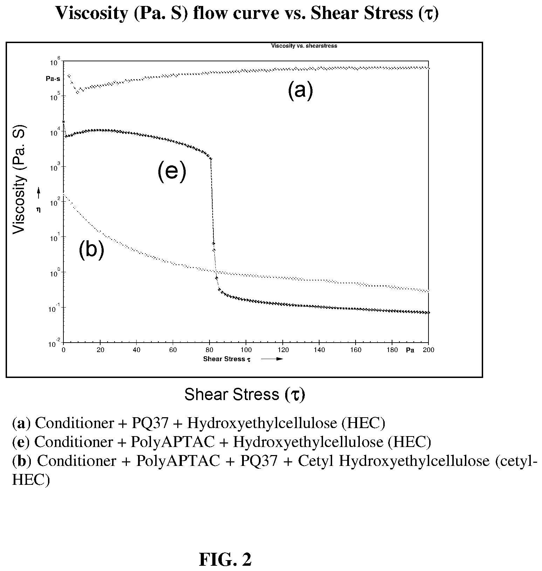

The PolyAPTAC polymer was tested as a 1 wt% in 2.5% fatty alcohol and 0.7% Cationic surfactants (Example "**b**" of Table 1). As a reference 1 wt. % Polyquaternium-37 in 2.5% fatty alcohol and 0.7% Cationic surfactants was used (Example "**a**" of Table 1). Example "**e**" of Table 1, consists of 0.25 wt. % PolyAPTAC polymer and 0.25 wt. % Polyquaternium-37 polymer in 2.5% fatty alcohol and 0.7% Cationic surfactants and 0.1 wt. % Natrosol Plus 330. Rheology profile Viscosity *vs*. Shear stress of above mentioned examples was measured with Anton Paar Rheometer. The results are provided in **Figure 2****.**

From the results presented in **Figure 2****,** it is evident that a PolyAPTAC polymer when used in combination with Polyquaternium-37 and hydrophobically modified Cellulosics such as Natrosol Plus 330 provides (example "e") better rheology profile compared to the conditioning polymers alone.

## Claims

1. A personal care conditioning and/or styling synergistic composition for a keratin substrate comprising:
i. 0.1 wt. % to 20 wt. % of poly(acrylamido-N-propyltrimethylammonium chloride) (PolyAPTAC) polymer, wherein
- the average molecular weight of PolyAPTAC is in the range of from 100,000 to 1,000,000 g/mol, and
- the PolyAPTAC polymer has a cationic charge density of 1 meq/g to 8 meq/g;
ii. 0.1 wt. %to 5 wt. % of Polyquaternium-37;
iii. 0.1 wt. % to 5 wt. % of at least one cationic surfactants;
iv. 0.1 wt. % to 99.59 wt. % of water; and
v. 0.01 wt. % to 20 wt. % of at least one cosmetically acceptable excipient,
wherein the composition is capable of providing immediate and prolonged benefit to a desired keratin substrate which is hair or skin;
with the proviso that the following compositions are excluded:
| | Gew.-% | |
|---|---|---|
| Cetearyl Alcohol | 7,0 | 7,0 |
| Quaternium-87 | 0,9 | 0,9 |
| Glycol Distearate | 1,0 | 1,0 |
| Poly(3-Acrylamidopropyl)trimethylammoniumchlorid* | 1,0 | 1,3 |
| Citronensäure | 1,5 | 1,5 |
| Methyl-N,N-bis(stearoyloxyethyl)-N-(2-hydroxyethyl)ammonium-methosulfat | 2,0 | 2,0 |
| Polyquaternium-10 | 0,1 | 0,1 |
| Baobab Seed Oil (INCI: Adansonia digitata Seed Oil) | 0,1 | 0,1 |
| Cetrimonium Chloride | 3,5 | 3,5 |
| Polyquaternium-37 | 0,4 | 0,4 |
| Dimethicone | 1,0 | - |
| Wasser, Konservierung, Begleitstoffe und ggf. Parfümöle | ad 100 | ad 100 |
| | | |
|---|---|---|
| * N-DurHance A-1000 Ashland Inc. Unless otherwise stated, all quantities are by weight of active ingredient | | |

2. The composition according to claim 1, wherein said cosmetically acceptable excipient is selected from the group consisting of fatty substances, gelling agents, thickeners, surfactants, moisturizers, emollients, hydrophilic or lipophilic active agent, antioxidants, sequestering agents, preserving agents, acidifying or basifying agents, fragrances, fillers, dyestuffs, emulsifying agents, solvents, UV-A or UV-B blocker/filters, plant extracts, moisturizers, proteins, peptides, neutralizing agents, film forming agents, setting agents, wax materials, oils, solvents, silicones and/or reducing agents.

3. The composition according to claim 1, wherein the pH of said personal care compositions is in the range of from 3 to 13.

4. The composition according to claim 1, wherein said personal care composition is an appropriate product selected from the group consisting of hair-care products, shampoos, hair conditioners, leave in and rinse off conditioners, styling and treating hair compositions, hair perming products, hair relaxants, hair straighteners, hair sprays and lacquers, permanent hair dyeing systems, hair styling mousses, hair gels, semi-permanent hair dyeing systems, temporary hair dyeing systems, hair bleaching systems, permanent hair wave systems, hair setting formulations, skin-care products, bath products, shower products, liquid soaps, bar soaps, fragrances and/or odoriferous ingredients consisting preparations, dentifrices, deodorizing and antiperspirant preparations, decorative preparations, light protection formulations, shaving lotions, body oils, body lotions, body gels, treatment creams, body cleaning products, skin protection ointments, shaving and aftershave preparations, skin powders, lipsticks, nail varnishes, eye shadows, mascaras, dry and moist make-up, rouge, powders, depilatory agents, sun care products, and/or compositions comprising UV blockers or UV protectors.

5. The composition according to claim 1, wherein said composition is formulated as an emulsion, a lotion, a gel, a vesicle dispersion, a paste, a cream, a solid stick, a mousse, a shampoo, powder, and/or a spray.

6. The composition according to claim 1, wherein the effective amount of composition used in the personal care composition is in the range of from 0.01 wt. % to 5.0 wt. %.

7. A method for treating or fixing regular or damaged keratin substrate comprising contacting said keratin substrate with an effective amount of personal care composition of claim 1.

8. A method for washing or caring a keratin substrate comprising applying an effective amount of composition of claim 1.

## Patentansprüche

1. Synergistische Körperpflegespülungs- und/oder Körperpflegestylingzusammensetzung für ein Keratinmaterial umfassend:
i. 0,1 Gew.-% bis 20 Gew.-% Poly(acrylamido-N-propyltrimethylammonium-chlorid) (PolyAPTAC)-Polymer, wobei
- wobei das durchschnittliche Molekulargewicht von PolyAPTAC im Bereich von 100.000 bis 1.000.000 g/mol liegt, und
- das PolyAPTAC eine kationische Ladungsdichte von 1 meq/g bis 8 meq/g aufweist;
ii. 0,1 Gew.-%bis 5 Gew.-% Polyquaternium-37;
iii. 0,1 Gew.-% bis 5 Gew.-% wenigstens eines kationischen Tensids;
iv. 0,1 Gew.-% bis 99,59 Gew.-% Wasser; und
v. 0,01 Gew.-% bis 20 Gew.-% wenigstens eines kosmetisch akzeptablen Hilfsstoff;
wobei die Zusammensetzung in der Lage ist, einem gewünschten Keratinmaterial, bei dem es sich um Haar oder Haut handelt, einen sofortigen und anhaltenden Nutzen zu verleihen;
mit der Maßgabe, dass die folgenden Zusammensetzungen ausgeschlossen sind:
| | Gew.-% | |
|---|---|---|
| Cetearylalkohol | 7,0 | 7,0 |
| Quaternium-87 | 0,9 | 0,9 |
| Glykol-Distearat | 1,0 | 1,0 |
| Poly(3-Acrylamidopropyl)trimethylammoniumchlorid* | 1,0 | 1,3 |
| Citronensäure | 1,5 | 1,5 |
| Methyl-N,N-bis(stearoyloxyethyl)-N-(2-hydroxyethyl)ammonium-methosulfat | 2,0 | 2,0 |
| Polyquaternium-10 | 0,1 | 0,1 |
| Baobab-Saatöl (INCI: Adansonia digitata Samenöl) | 0,1 | 0,1 |
| Cetrimoniumchlorid | 3,5 | 3,5 |
| Polyquaternium-37 | 0,4 | 0,4 |
| Dimethicone | 1,0 | -- |
| Wasser, Konservierung, Begleitstoffe und ggf. Parfümöle | auf 100 | auf 100 |
| | | |
|---|---|---|
| * N-DurHance A-1000 Ashland Inc. Sofern nicht anders angegeben, beziehen sich alle Mengen auf das Gewicht des Inhaltsstoffs. | | |

2. Die Zusammensetzung gemäß Anspruch 1, wobei der kosmetisch akzeptable Hilfsstoff ausgewählt wird aus der Gruppe bestehend aus Fettsubstanzen, Geliermitteln, Verdickungsmitteln, Tensiden, Feuchthaltemitteln, Emollienzien, hydrophilen oder lipophilen Wirkstoffen, Antioxidantien, Sequestriermitteln, Konservierungsmitteln, säurebildende oder basenbildende Mitteln, Duftstoffen, Füllstoffen, Farbstoffen, Emulgatoren, Lösungsmitteln, UV-A- oder UV-B-Blockern/Filtern, Pflanzenextrakten, Feuchthaltemitteln, Proteinen, Peptiden, Neutralisationsmitteln, Filmbildnern, Fixiermitteln, Wachsmaterialien, Ölen, Lösungsmitteln, Silikonen, und Reduktionsmitteln.

3. Die Zusammensetzung gemäß Anspruch 1, wobei der pH-Wert der Körperpflegezusammensetzungen im Bereich von 3 bis 13 liegt.

4. Die Zusammensetzung gemäß Anspruch 1, wobei die Körperpflegezusammensetzung ein geeignetes Produkt ist, das ausgewählt wird aus der Gruppe bestehend aus Haarpflegeprodukten, Shampoos, Haarspülungen, Leave-in- und Rinse-off-Conditionern, Styling- und Behandlungshaarzusammensetzungen, Dauerwellprodukten, Haarlockerungsmitteln, Haarglättmitteln, Haarsprays und - lacken, permanenten Haarfärbesystemen, Haar-Styling-Schäumen, Haargelen, semipermanenten Haarfärbesystemen, temporären Haarfärbesystemen, Haarbleichungssystemen, permanenten Haarwellensystemen, Haarfestigungsformulierungen, Hautpflegeprodukten, Badeprodukten, Duschprodukten, Flüssigseifen, Seifenstücken, Zubereitungen bestehend aus Duftstoffen und/oder wohlriechenden Inhaltsstoffen, Zahnputzmitteln, desodorierenden und schweißhemmenden Präparaten, dekorativen Präparate, Lichtschutzformulierungen, Rasierwassern, Körperölen, Körperlotionen, Körpergelen, Behandlungscremen, Körperreinigungsprodukten, Hautschutzsalben, Rasiermitteln und Aftershaves, Hautpuder, Lippenstiften, Nagellacke, Lidschatten, Mascaras, trockenes und feuchtes Make-up, Rouge, Pudern, Enthaarungsmitteln, Sonnenschutzmitteln und/oder Zusammensetzungen, die UV-Blocker oder UV-Schutzmittel enthalten.

5. Die Zusammensetzung gemäß Anspruch 1, wobei die Zusammensetzung als Emulsion, Lotion, Gel, Vesikeldispersion, Paste, Creme, fester Stift, Mousse, Shampoo, Puder und/oder Spray formuliert ist.

6. Die Zusammensetzung gemäß Anspruch 1, wobei die wirksame Menge der in der Körperpflegezusammensetzung verwendeten Zusammensetzung im Bereich von 0,01 Gew.-% bis 5,0 Gew.-% liegt.

7. Ein Verfahren zur Behandlung oder Fixierung von regulärem oder beschädigtem Keratinmaterial, umfassend das Inkontaktbringen des Keratinmaterials mit einer wirksamen Menge einer Körperpflegezusammensetzung gemäß Anspruch 1.

8. Ein Verfahren zum Waschen oder Pflegen eines Keratinmaterials, umfassend das Auftragen einer wirksamen Menge einer Zusammensetzung gemäß Anspruch 1.

## Revendications

1. Composition synergique de conditionnement et/ou de coiffage destinée à des soins personnels, pour un substrat kératinique, comprenant :
i. de 0,1% en poids à 20% en poids d'un polymère de poly(chlorure d'acrylamido-N-propyltriméthylammonium) (PolyAPTAC), où
- le poids moléculaire moyen du PolyAPTAC se trouve dans la plage allant de 100 000 à 1 000 000 g/mol, et
- le polymère de PolyAPTAC possède une densité de charge cationique allant de 1 méq./g à 8 méq./g,
ii. de 0,1% en poids à 5% en poids de polyquaternium-37 ;
iii. de 0,1% en poids à 5% en poids d'au moins un agent tensioactif cationique ;
iv. de 0,1% en poids à 99,59% en poids d'eau ; et
v. de 0,01% en poids à 20% en poids d'au moins un excipient cosmétiquement acceptable ;
la composition étant capable d'apporter un bénéfice immédiat et prolongé à un substrat kératinique désiré qui est constitué de cheveux ou de peau ;
à condition que les compositions suivantes soient exclues :
| | Gew.-% | |
|---|---|---|
| Alcool cétéarylique | 7,0 | 7,0 |
| Quaternium-87 | 0,9 | 0,9 |
| Distéarate de glycérol | 1,0 | 1,0 |
| Poly(3-Acrylamidopropyl)trimethylammoniumchlorid* | 1,0 | 1,3 |
| Citronensäure | 1,5 | 1,5 |
| Methyl-N,N-bis(stearoyloxyethyl)-N-(2-hydroxyethyl)ammonium-methosulfat | 2,0 | 2,0 |
| Polyquaternium-10 | 0,1 | 0,1 |
| Huile de graines de Baobab (INCI : Huile de graines d'*Adansonia digitata*) | 0,1 | 0,1 |
| Chlorure de cétrimonium | 3,5 | 3,5 |
| Polyquaternium-37 | 0,4 | 0,4 |
| Diméthicone | 1,0 | - |
| Wasser, Konservierung, Begleitstoffe und ggf. Parfümöle | qsp 100 | qsp 100 |
| | | |
|---|---|---|
| *N-DurHance A-1000, Ashland Inc. Sauf indication contraire, toutes les quantités sont en poids d'ingrédient actif. | | |

2. Composition selon la revendication 1, dans laquelle ledit excipient cosmétiquement acceptable est choisi dans le groupe constitué par les substances grasses, les agents gélifiants, les épaississants, les agents tensioactifs, les hydratants, les émollients, les agents actifs hydrophiles ou lipophiles, les antioxydants, les agents séquestrants, les conservateurs, les agents acidifiants ou alcalinisants, les fragrances, les charges, les matières colorantes, les agents émulsifiantes, les solvants, les filtres anti-UVA ou UVB/agents bloquant les UVA ou UVB, les extraits végétaux, les hydratants, les protéines, les peptides, les agents neutralisants, les agents filmogènes, les agents fixants, les matériaux à base de cire, les huiles, les solvants, les silicones, et/ou les agents réducteurs.

3. Composition selon la revendication 1, dans laquelle le pH desdites compositions de soins personnels se trouve dans la plage allant de 3 à 13.

4. Composition selon la revendication 1, ladite composition de soins personnels étant un produit approprié choisi dans le groupe constitué par les produits de soin capillaire, les shampooings, les après-shampooings, les après-shampooings avec et sans rinçage, les compositions capillaires de coiffage et de traitement, les produits pour permanentes capillaires, les défrisants capillaires, les lissants capillaires, les sprays capillaires et les laques, les systèmes de teinture capillaire permanente, les mousses de coiffage capillaires, les gels capillaires, les systèmes de teinture capillaire semi-permanente, les systèmes de teinture capillaire temporaire, les systèmes de décoloration capillaire, les systèmes d'ondulation capillaire permanente, les formulations de fixage capillaire, les produits de soin de la peau, les produits pour le bain, les produits pour la douche, les savons liquides, les savons en pain, les parfums et/ou les préparations constituées d'ingrédients odorifères, les dentifrices, les préparations désodorisantes et antitranspirantes, les préparations décoratives, les formulations de photoprotection, les lotions de rasage, les huiles pour le corps, les lotions pour le corps, les gels pour le corps, les crèmes traitantes, les produits de nettoyage du corps, les onguents de protection de la peau, les préparations de rasage et d'après-rasage, les poudres pour la peau, les rouges à lèvres, les vernis à ongles, les fards à paupières, les mascaras, les maquillages secs et humides, le fard à joues, les poudres, les agents épilatoires, les produits de soin solaire, et/ou les compositions comprenant des agents bloquant les UV ou des agents de protection contre les UV.

5. Composition selon la revendication 1, ladite composition étant formulée sous forme d'une émulsion, d'une lotion, d'un gel, d'une dispersion de vésicules, d'une pâte, d'une crème, d'un bâtonnet solide, d'une mousse, d'un shampooing, d'une poudre et/ou d'un spray.

6. Composition selon la revendication 1, dans laquelle la quantité efficace de composition utilisée dans la composition de soins personnels se trouve dans la plage allant de 0,01% en poids à 5,0% en poids.

7. Méthode de traitement ou de fixage d'un substrat kératinique ordinaire ou endommagé, comprenant la mise en contact dudit substrat kératinique avec une quantité efficace d'une composition de soins personnels selon la revendication 1.

8. Méthode de lavage ou de soin d'un substrat kératinique, comprenant l'application d'une quantité efficace d'une composition selon la revendication 1.
